# DEMANDE DE BREVET EUROPEEN

(11) **EP 1 240 888 A1**
(43) Date de publication de la demande: **18.09.2002**
(21) Numéro de dépôt: 02290465.0
(22) Date de dépôt: 26.02.2002
(51) Int. Cl.: A61K 7/06, A61K 7/48

(54) **Composition capillaire comprenant un polysaccharide greffé par un polysiloxane et un polymére fixant**

(30) Priorité: 13.03.2001 FR 0103404
(71) Demandeur: L'OREAL, 75008 Paris (FR)
(72) Inventeur: Giroud, Franck, 92110 Clichy (FR); Dupuis, Christine, 75018 Paris (FR)
(74) Mandataire: Bourdeau, Françoise

(57) **Abrégé**

L'invention a pour objet une composition cosmétique capillaire comprenant au moins un polymère polysaccharide greffé par un polysiloxane et au moins un polymère fixant non siliconé et non cellulosique. L'invention vise également un procédé cosmétique capillaire comprenant l'application de cette composition sur les cheveux ainsi que son utilisation pour fixer et/ou maintenir la coiffure.

## Description

L'invention a pour objet une composition cosmétique capillaire comprenant au moins un polymère polysaccharide greffé par un polysiloxane et au moins un polymère fixant non siliconé et non cellulosique. L'invention vise également un procédé cosmétique capillaire comprenant l'application de cette composition sur les cheveux ainsi que son utilisation pour fixer et/ou maintenir la coiffure.

Parmi les produits capillaires, notamment ceux destinés à la mise en forme et/ou au maintien de la coiffure les plus répandus sur le marché de la cosmétique, on distingue les compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en forme de la coiffure. Ces gels ou mousses peuvent également contenir des résines polymères.

Toutefois, ces compositions capillaires présentent souvent l'inconvénient d'altérer les propriétés cosmétiques des cheveux: Ainsi, les cheveux peuvent devenir rêches, difficiles à démêler, perdre leur toucher et leur aspect agréables ou encore manquer de corps. Par ailleurs, les compositions capillaires peuvent donner lieu à un effet de poudrage, donnant à l'utilisateur un aspect négligé.

Il existe donc un besoin de trouver des compositions cosmétiques, notamment pour le coiffage, qui ne présentent pas l'ensemble des inconvénients indiqués ci-dessus et qui, en particulier, fixent bien la coiffure tout en procurant de bonnes propriétés cosmétiques, comme un toucher agréable ou un aspect naturel.

De manière surprenante et inattendue, la Demanderesse a découvert que lorsque l'on associe des composés spécifiques, à savoir des polysaccharides greffés par des polysiloxanes avec certains polymères fixants sélectionnés, il est possible d'obtenir des compositions cosmétiques répondant aux exigences exprimées ci-dessus.

L'invention a pour objet une composition cosmétique comprenant, dans un milieu cosmétiquement acceptable, au moins un polymère à squelette de type polysaccharide greffé par des chaînons contenant au moins un polysiloxane et au moins un polymère fixant non siliconé et non cellulosique.

Un autre objet de la présente invention concerne un procédé cosmétique, notamment capillaire, comprenant l'application d'une composition conforme à l'invention.

Encore un autre objet de l'invention concerne l'utilisation de cette composition dans une formulation cosmétique capillaire, destinée notamment au maintien et/ou à la mise en forme de la coiffure.

Les polymères à squelettes de type polysaccharide greffés par des chaînons contenant au moins un polysiloxane conformes à la présente invention comprennent une chaîne principale formée à partir de polysacharide(s), sur laquelle se trouve greffé, ainsi qu'éventuellement à l'une au moins de ses extrémités, au moins un chaînon contenant un polysiloxane.

Les polymères à squelettes de type polysaccharide greffés par des chaînons contenant au moins un polysiloxane conformes à la présente invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs polysaccharide(s), eux-mêmes correctement fonctionnalisés par une fonction, capable de venir réagir avec le ou les groupements fonctionnels portés par ladite silicone pour former une liaison covalente.

Les polymères à squelettes polysaccharide greffés par des chaînons contenant au moins un polysiloxane conformes à l'invention sont choisis, de préférence, parmi ceux décrits dans le brevet US 6 066 727 déposé par la Société Shin-Etsu. Il s'agit de copolymères obtenus par réaction entre un polysaccharide contenant des groupements carboxyles et un polysiloxane contenant un groupement époxy terminal, dans un solvant organique, en présence éventuellement d'un catalyseur.

En particulier, les polymères à squelettes polysaccharide greffés par des chaînons contenant au moins un polysiloxane utilisés plus préférentiellement conformément à la présente invention sont susceptibles d'être obtenus conformément au procédé décrit dans le brevet US 6 066 727, c'est-à-dire en :
(a) mettant en solution, dans un solvant organique, un polysaccharide contenant des groupements carboxyles et un polysiloxane, contenant lui-même un groupement époxy terminal, et répondant à la formule (I) de polysiloxane suivante : 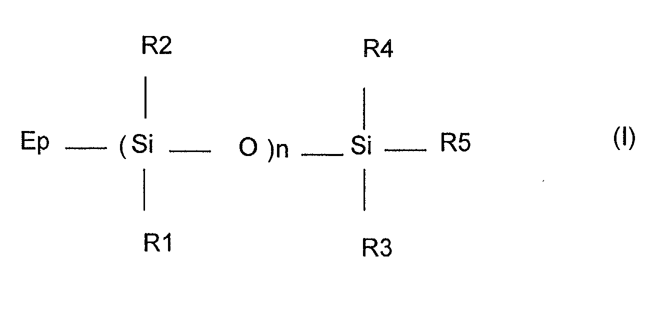 dans laquelle :
   - n est un nombre entier compris entre 3 à 500,
   - R₁, R₂, R₃, R₄ et R₅ sont choisis, indépendamment l'un de l'autre, parmi les hydrocarbures monovalents en C₁ à C₁₀ ou les hydrocarbures halogénés monovalents en C₁ à C₁₀, et
   - Ep est le 2-(3,4-époxycyclohexyl) éthyl ; et
(b) chauffant ce mélange de polysaccharides et de polysiloxanes à une température comprise entre 60 et 200 °C, de façon à faire réagir les groupements carboxyles des polysaccharides avec les groupements époxy des polysiloxanes.

Parmi des polysaccharides convenant particulièrement pour la mise en oeuvre de l'étape (a) de ce procédé, on peut citer les polysaccharides possédant des groupements carboxylique, benzoyle ou succinoyle, tels que l'hydroxypropyl méthyl cellulose phtalate, l'hydroxypropyl méthyl cellulose acétate succinate, le carboxyméthyl éthyl cellulose, le polysaccharide pullulan acétate phthalate. On préfère, notamment, l'hydroxypropyl méthyl cellulose phthalate et l'hydroxypropyl méthyl cellulose acétate succinate.

De manière avantageuse, R₁, R₂, R₃, R₄ et R₅ sont choisis, indépendamment l'un de l'autre, parmi les groupements méthyl, éthyl, propyl, butyl, cycloalkyl en C₃ à C₈, tels que les radicaux cyclopentyl ou cyclohexyl, aryles, tels que les radicaux phényl et le tolyl, aralkyls en C₃ à C₈, tels que les radicaux benzyl et phénéthyl et alcényl, tels que les groupes vinyl et allyl. Ces radicaux hydrocarbyls monovalents peuvent, éventuellement, être totalement ou partiellement substitués, en particulier, par un atome d'halogène, comme les radicaux chlorométhyl et 3,3,3-trifluoropropyl.

En tant que solvants organiques convenant spécialement bien pour la réalisation de l'étape (a) du procédé, on peut citer les cétones, comme l'acétone ou la cyclohexanone.

L'étape (b) est généralement effectuée sous agitation, éventuellement sous atmosphère inerte.

La composition comprend avantageusement, en pourcentage relatif en poids de la composition, de 0,1 à 20 % de polysaccharides greffés par des polysiloxanes, et plus préférentiellement de 0,5 à 10 % de ce polysaccharide.

La composition comprend avantageusement, en pourcentage relatif en poids de la composition, de 0,01 à 20 % de polymère fixant, et de préférence, de 0,05 à 10 % .

Les polymères fixants non siliconés et non cellulosiques convenant dans l'invention sont ceux généralement utilisés dans la technique. Ils sont notamment choisis parmi les polymères cationiques, anioniques, amphotères, non ioniques et leurs mélanges.

Les polymères fixants non siliconés et non cellulosiques peuvent être solubles dans le milieu cosmétiquement acceptable ou insolubles dans ce même milieu et utilisés dans ce cas sous forme de dispersions de particules solides ou liquides de polymère (latex ou pseudolatex).

On entend par *« polymère fixant non cellulosique »* tout polymère fixant exempt de motif cellobiose.

On entend par « *polymère fixant non siliconé »* tout polymère fixant exempt d'atome de silicium dans sa structure.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs de formules suivantes : 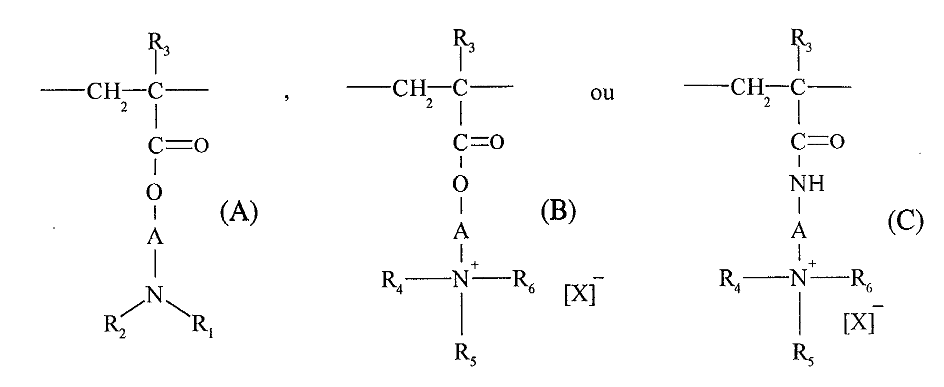 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle,
   - les copolymères d'acrylamide et de chlorure de méthacryloyloxyéthyltriméthylammonium décrits, par exemple, dans la demande de brevet EP-A-080976,
   - les copolymères d'acrylamide et de méthosulfate de méthacryloyloxyéthyltriméthylammonium,
   - les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français 2 077 143 et 2 393 573,
   - les terpolymères méthacrylate de diméthylaminoéthyle/vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination "GAFQUAT® HS 100" par la société ISP.
(2) les gommes de guar quaternisées;
(3) les copolymères quaternaires de vinylpyrrolidone et de vinylimidazole ;
(4) les chitosanes ou leurs sels ;
les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 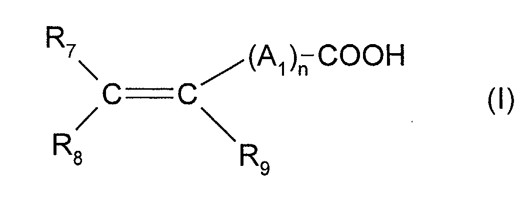 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylèneglycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique □- ou □-cyclique. De tels polymères sont décrits entre autres dans les brevets français n^{os} 1 222 944, 1 580 545, 2 265 782, 2 265 781, 1 564 110 et 2 439 798. Un produit commercial entrant dans cette classe est la résine 28-29-30 commercialisée par la société National Starch.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, alpha-oléfine, esters acryliques ou méthacryliques, acides acrylique ou méthacrylique ou vinylpyrrolidone dans leur chaîne,
      les fonctions anhydrides de ces copolymères étant éventuellement monoestérifiées ou monoamidifiées.
      Ces polymères sont par exemple décrits dans les brevets français n^{os} 2 350 384 et 2 357 241 de la demanderesse.
E) Les polyacrylamides comportant des groupements carboxylates ;
F) les polyuréthannes anioniques, tels que le produit commercialisé par BASF sous la dénomination Luviset PUR.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous la dénomination Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertiobutylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF, les copolymères acétate de vinyle/acide crotonique et les copolymères acétate de vinyle/acide crotonique greffés par du polyéthylèneglycol vendus sous la dénomination ARISTOFLEX® A par la société BASF ainsi que le polyuréthanne Luviset PUR® commercialisé par la Société BASF.

Les polymères fixants anioniques les plus particulièrement préférés sont choisis parmi les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP ainsi que le polyuréthanne Luviset PUR® commercialisé par la Société BASF.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-alpha-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diéthyldiallylammonium. Les copolymères d'acide acrylique et de ce dernier monomère sont proposés sous les appelations MERQUAT 280, MERQUAT 295 et MERQUAT PLUS 3330 par la société CALGON.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale : 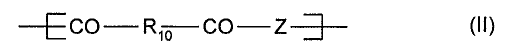 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 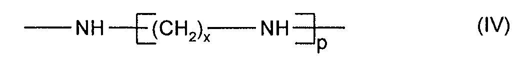 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 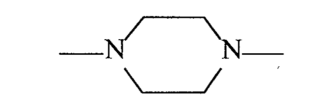
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 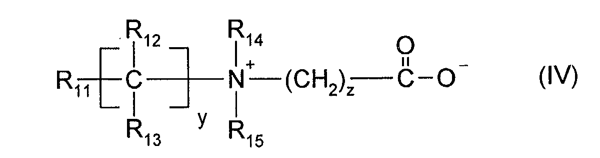 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de butyle/méthacrylate de N,N-diméthylcarboxyaminoéthyl.
(5) les polymères dérivés du chitosane comportant des motifs monomères répondant aux formules suivantes : 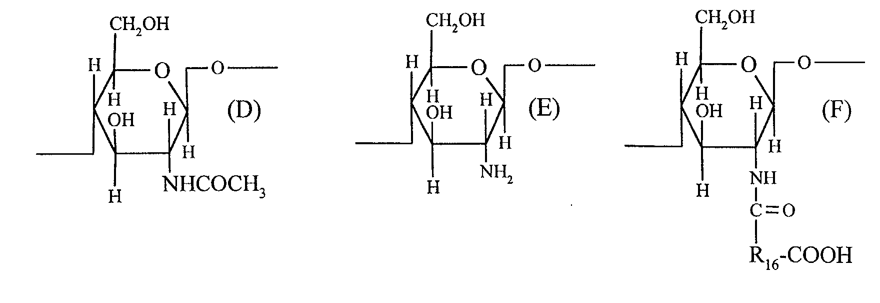 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 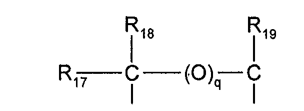 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 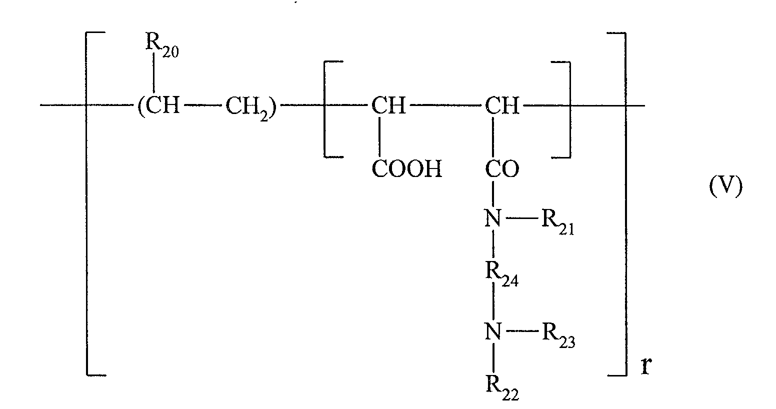 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule :-R₂₄-N(R₂₂)₂, R₂₄ repré-sentant un groupement -CH₂-CH₂- , -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane.
(8) Les polymères amphotères du type -D-X-D-X choisis parmi:
   a) les polymères obtenus par action de l'acide chloracétique ou le chloracétate de sodium sur les composés comportant au moins un motif de formule:

      -D-X-D-X-D- (VI)

      où D désigne un groupe 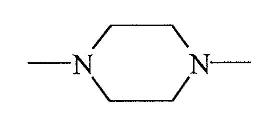 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée
      ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')
   où D désigne un groupe 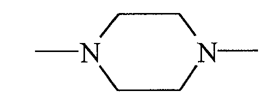 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de butyle/méthacrylate de N,N-diméthylcarboxyaminoéthyl.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS;
- les homopolymères de styrène ;
- les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ;
- les copolymères de styrène, de méthacrylate d'alkyle et d'acrylate d'alkyle
- les polyuréthannes non ioniques,
- les copolymères de styrène et de butadiène ;
- les copolymères de styrène, de butadiène et de vinylpyridine;
- les copolymères d'acrylate d'alkyle et d'uréthanne ;
- les polyamides,
- les homopolymères et copolymères de vinyllactame.

Les groupes alkyle des polymères non ioniques mentionnés ci-dessus ont, de préférence, de 1 à 6 atomes de carbone.

Selon la présente invention, les polymères fixants sont de préférence des polymères non-ioniques, et mieux encore des polymères non ioniques à motifs vinyllactames. Ils sont notamment décrits dans les brevets US 3 770 683, US 3 929 735, US 4 521 504, US 5 158 762, US 5 506 315 et dans les demandes de brevet WO 94/121148, WO 96/06592 et WO 96/10593. Ils peuvent se présenter sous forme pulvérulente ou sous forme de solution ou de suspension.
Les homopolymères ou copolymères à motifs vinyllactame comprennent des motifs de formule : 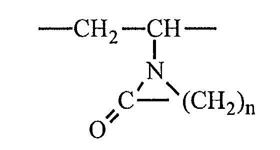 dans laquelle n est indépendamment 3, 4 ou 5.

La masse moléculaire en nombre des polymères à motifs vinyllactames est généralement supérieure à environ 5 000, de préférence comprise entre 10 000 et 1 000 000 environ, plus préférentiellement comprise entre 10 000 et 100 000 environ.

On peut utiliser, en particulier, comme polymère fixant, dans la présente invention, les polyvinylpyrrolidones telles que celles commercialisées sous la dénomination Luviskol® K30 par la société BASF ; les polyvinylcaprolactames tels que ceux commercialisés sous la dénomination Luviskol® PLUS par la société BASF ; les copolymères poly(vinylpyrrolidone/acétate de vinyle) tels que ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) tels que, par exemple, ceux commercialisés sous la dénomination Luviskol® VAP 343 par la société BASF.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄, des cétones aliphatiques ou aromatiques, des esters d'acides à chaînes courtes en C₁ à C₈ ou longues et C₉ à C₂₀ et d'alcools à chaînes courtes C₁ à C₈ ou longues C₉ à C₂₀, le pentane, l'heptane, les polyols, les éthers de polyol et l'isodécane.

Parmi les alcools, on peut citer l'éthanol, l'isopropanol. L'éthanol est particulièrement préféré.

Le rapport entre la concentration relative en poids des polymères à squelettes de type polysaccharide et la concentration relative en poids des polymères fixants, dans les compositions conformes à l'invention, est avantageusement compris entre 0,01 et 100, plus avantageusement entre 0,05 et 20, et plus avantageusement encore entre 0,1 et 10.

La composition de l'invention peut également contenir au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'inventions peuvent être appliquées sur la peau, les ongles, les lèvres, les cheveux, les sourcils et les cils.

Les compositions conformes à l'invention sont particulièrement adaptées pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLE :

On réalise la composition suivante conforme à l'invention.

| Composition 1: | |
|---|---|
| Cellulose à greffons silicone [1] | 3% |
| Ultrahold Strong [2] | 3% |
| Eau | 10% |
| Ethanol qsp | 100% |

| | |
|---|---|
| [1] Hydroxy proplyl methyl cellulose acétate succinate à greffons polydiméthylsiloxane, telle qu'elle est synthétisée dans l'exemple N°2 du brevet US 6,066,727 de la société Shin Etsu. | |
| [2] acrylates/butylacrylamide copolymère commercialisé par la Société BASF | |

La composition est appliquée sur des mèches de cheveux eurochâtains. Elle donne de très bons résultats en pouvoir laquant et en cosmétique, notamment en démêlage, douceur et toucher, tout en donnant lieu à un faible poudrage.

## Revendications

1. Composition cosmétique comprenant, dans un milieu cosmétiquement acceptable, au moins un polymère à squelette de type polysaccharide greffé par des chaînons contenant au moins un polysiloxane et au moins un polymère fixant non siliconé et non cellulosique.

2. Composition selon la revendication 1, **caractérisée par le fait que** les polymères à squelettes de type polysaccharide greffés par des chaînons contenant au moins un polysiloxane comprennent une chaîne principale formée à partir de polysacharide(s), sur laquelle se trouve greffé, ainsi qu'éventuellement à l'une au moins de ses extrémités, au moins un chaînon contenant un polysiloxane.

3. Composition selon l'une quelconque des revendications précédentes, **caractérisée par le fait que** les que les polymères à squelettes de type polysaccharide greffés par des chaînons contenant au moins un polysiloxane sont susceptibles d'être obtenus en :
(a) mettant en solution, dans un solvant organique, un polysaccharide contenant des groupements carboxyles et un polysiloxane, contenant lui-même un groupement époxy terminal, et répondant à la formule (I) de polysiloxane suivante : 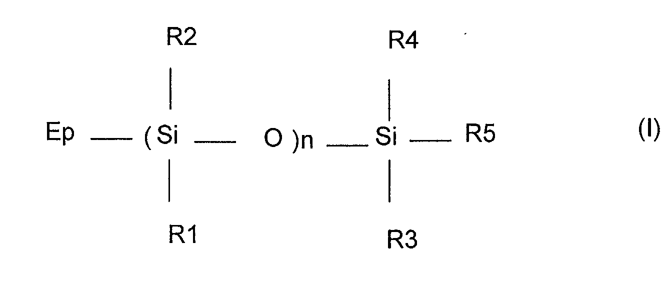 dans laquelle :
- n est un nombre entier compris entre 3 à 500,
- R₁, R₂, R₃, R₄ et R₅ sont choisis, indépendamment l'un de l'autre, parmi les hydrocarbures monovalents en C₁ à C₁₀ ou les hydrocarbures halogénés monovalents en C₁ à C₁₀, et
- Ep est le 2-(3,4-époxycyclohexyl) éthyl ; et
(b) chauffant ce mélange de polysaccharides et de polysiloxanes à une température comprise entre 60 et 200 °C, de façon à faire réagir les groupements carboxyles des polysaccharides avec les groupements époxy des polysiloxanes.

4. Composition selon la revendication 3, **caractérisée par le fait que** pour la mise en oeuvre de l'étape (a), on utilise, en tant que polysaccharides, des polysaccharides possédant des groupements carboxylique, benzoyle ou succinoyle, tels que l'hydroxypropyl méthyl cellulose phtalalate, l'hydroxypropyl méthyl cellulose acétate succinate, le carboxyméthyl éthyl cellulose, le polysaccharide pullulan acétate phthalate.

5. Composition selon la revendication 3, **caractérisée par le fait que** R₁, R₂, R₃, R₄ et R₅ sont choisis, indépendamment l'un de l'autre, parmi les groupements méthyl, éthyl, propyl, butyl, cycloalkyl en C₃ à C₈, aryls, aralkyls en C₃ à C₈ et alcényl.

6. Composition selon l'une quelconque des revendications précédentes, **caractérisée par le fait que** les polymères fixants sont choisis parmi les polymères cationiques, anioniques, amphotères, non ioniques et leurs mélanges.

7. Composition selon la revendication 6, **caractérisée par le fait que** le polymère fixant est un polymère fixant anionique à groupements carboxyliques choisi parmi :
A) Les homo- ou copolymères d'acide acrylique ou méthacrylique ou leurs sels, les copolymères d'acide acrylique et d'acrylamide, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylèneglycol tel que le polyéthylène-glycol, et éventuellement réticulés ;
C) Les copolymères dérivés d'acide crotonique ;
D) Les copolymères dérivés d'acides ou d'anhydrides carboxyliques monoinsaturés en C₄-C₈ choisis parmi :
- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ;
- les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, alpha-oléfine, esters acryliques ou méthacryliques, acides acrylique ou méthacrylique ou vinylpyrrolidone dans leur chaîne ;
E) Les polyacrylamides comportant des groupements carboxylates ;
F) les polyuréthannes anioniques.

8. Composition selon la revendication 6, **caractérisée par le fait que** le polymère fixant est un polymère non ionique choisi parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques;
- les copolymères d'acrylonitrile et d'un monomère non ionique;
- les homopolymères de styrène ;
- les copolymères de styrène et de (méth)acrylate d'alkyle;
- les copolymères de styrène, de méthacrylate d'alkyle et d'acrylate d'alkyle;
- les polyuréthannes non ioniques,
- les copolymères de styrène et de butadiène ;
- les copolymères de styrène, de butadiène et de vinyipyridine ;
- les copolymères d'acrylate d'alkyle et d'uréthanne ;
- les polyamides,
- les homopolymères et copolymères de vinyllactame.

9. Composition selon l'une quelconque des revendications précédentes, **caractérisée par le fait que** le rapport entre la concentration relative en poids des polymères à squelettes de type polysaccharide et la concentration relative en poids des polymères fixants, dans les compositions conformes à l'invention, est compris entre 0,01 et 100, plus avantageusement entre 0,05 et 20, et plus avantageusement encore entre 0,1 et 10.

10. Composition selon l'une quelconque des revendications précédentes, **caractérisée par le fait que** la composition comprend, en pourcentage relatif en poids de la composition, de 0,1 à 20 % de polysaccharides greffés par des polysiloxanes, et plus préférentiellement de 0,5 à 10 % de ce polysaccharide.

11. Composition selon l'une quelconque des revendications précédentes, **caractérisée par le fait que** la composition comprend, en pourcentage relatif en poids de la composition, de 0,01 à 20 % de polymère fixant, et de préférence, de 0,05 à 10 % .

12. Composition selon l'une quelconque des revendications précédentes, **caractérisée par le fait qu'**elle contient au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

13. Procédé cosmétique capillaire, en particulier un procédé de fixation et/ou de maintien de la coiffure mettant en oeuvre une composition selon l'une quelconque des revendications 1 à 12.

14. Utilisation d'une composition selon l'une quelconque des revendications 1 à 12 dans une formulation cosmétique capillaire, destinée notamment au maintien et/ou à la mise en forme de la coiffure.
